# EUROPÄISCHE PATENTSCHRIFT

(11) **EP 0 663 180 B1**
(45) Veröffentlichungstag und Bekanntmachung des Hinweises auf die Patenterteilung: **03.03.1999**
(21) Anmeldenummer: 95100211.2
(22) Anmeldetag: 09.01.1995
(51) Int. Cl.: A61B 5/0215, G01L 9/00

(54) **Druckmesswandler zur Messung des Druckes einer Flüssigkeit, insbesondere zur invasiven Blutdruckmessung**
Pressure transducer for measuring the pressure of a liquid in particular for invasive blood pressure measurement
Transducteur de pression pour mesurer la pression d'un liquide en particulier pour mesure invasive de la pression artérielle

(30) Priorität: 14.01.1994 DE 4400941
(43) Veröffentlichungstag der Anmeldung: 19.07.1995
(73) Patentinhaber: PVB MEDIZINTECHNIK GmbH, 85614 Kirchseeon/Eglharting (DE)
(72) Erfinder: von Berg, Peter, Tiburon, CA 94920 (US)
(74) Vertreter: von Bülow, Tam, Dr.

(56) Entgegenhaltungen:
- EP-A- 0 124 308
- EP-A- 0 180 662
- EP-A- 0 330 011
- US-A- 4 679 567

## Beschreibung

Die Erfindung bezieht sich auf einen Druckmeßwandler gemäß dem Oberbegriff des Patentanspruches 1. Ein derartiger Druckmeßwandler ist aus der FR 2,125,991 oder aus der EP-A-330011 bekannt. Dort weist ein Gehäuse zwei durch eine Membran flüssigkeitsdicht getrennte Kammern auf, wobei die erste Kammer in Flüssigkeitsverbindung mit einer Zulauf- und einer Ablauföffnung steht. Die zweite Kammer enthält ein Druckmeßelement, das den von der Membran auf es übertragenen Flüssigkeitsdruck in elektrische Signale umwandelt. Diese Signale werden über elektrische Leitungen aus der zweiten Kammer nach außen geführt.

Es handelt sich hierbei um einen wiederverwendbaren Druckmeßwandler mit sogenanntem Druckdom, der nach Gebrauch abgeschraubt, gereinigt und sterilisiert werden kann und anschließend wiederverwendbar ist. Aufgrund des hohen Aufwandes für Reinigung und Sterilisierung werden seit Anfang der 90er Jahre in der Medizintechnik überwiegend nur noch einmal verwendbare Druckmeßwandler verwendet, die hinsichtlich Bedienung, Wartung etc. einfacher zu handhaben sind und das Infektionsrisiko für den Patienten drastisch reduzieren. Hierfür werden miniaturisierte Druckmeßelemente verwendet, die auf einem Keramiksubstrat befestigt sind, welches als gedruckte Schaltung auch die externe Beschaltung des Druckmeßelementes wzb. Abgleichwiderstände etc. und elektrische Leiterbahnen enthält. Ein solches Druckmeßelement ist in der US 4,023,562 beschrieben.

Solche Druckmeßelemente sind prinzipiell in Form einer Wheatstonschen Brückenschaltung mit einem Dehnungsmeßstreifen oder sonstigen, einen Druck oder mechanische Beanspruchungen in elektrische Singale umsetzenden Meßwandlern aufgebaut, wie sie der DE-OS 29 16 390 zu entnehmen ist.

Der erste Druckmeßwandler für die Blutdruckmessung, der mit solchen Druckmeßelementen arbeitet, ist in dem Firmenprospekt "Disposable Transducers" der Firma Cobe Laboratories Inc. mit Druckdatum 1983 beschrieben. Bei diesem Druckmeßwandler war das Keramiksubstrat mit dem Druckmeßelement in eine flüssigkeitsdicht abgeschlossene Druckmeßkammer eingesetzt, die mit einer Einlaß- und einer Auslaßöffnung in Flüssigkeitsverbindung stand. Die elektrischen Anschlüsse zur Abnahme der Meßwerte waren in Form von Leiterbahnen aus dieser Kammer seitlich herausgeführt und dort mit einem fest mit dem Gehäuse des Druckmeßwandlers verbundenem Kabel verlötet.

In der DE 29 46 515 A1 ist ein Druckgeber mit Hall-IC beschrieben, bei dem elektrische Leitungen des Druckmeßelementes zu fest im Gehäuse verankerten Steckkontakten führen, welche über eine Stecker-/Buchsenverbindung die elektrische Verbindung zu einem Kabel herstellen.

Ein weiterer Druckmeßwandler für die invasive Blutdruckmessung ist in der DE 2 07 044 A1 beschrieben, der ebenfalls als einmal verwandbares Gerät ausgebildet ist und bei dem ein Absperrhahn als Entnahmestelle für Blut zur Analyse integriert ist. Weiterhin ist dort vorgesehen, daß ein Kapillarsystem zum Schnellspülen in den Druckmeßwandler integriert ist.

In der Zeitschrift "Health Devices" Sept. 1983, S.268-290 sind in einem Aufsatz mit dem Titel "Disposable Pressure Transducers" verschiedene Druckmeßwandler für den einmaligen Gebrauch beschrieben, wobei einige dieser Druckmeßwandler gewisse Elektronikteile und Abgleichwiderstände in dem wiederverwendbaren Kabel untergebracht haben.

Die EP 0 124 308-B1 zeigt einen ähnlichen Druckmeßwandler, bei dem das Druckaufnehmerelement in einen elektrisch isolierenden Körper in Form einer Kapsel eingesetzt und der Kammer, die die zu messende Flüssigkeit enthält zugewandt angeordnet ist. Die elektrische Leitung erstreckt sich dabei durch den elektrisch isolierenden Körper hindurch und endet in Verbindungsmitteln im Gehäuse zur Herstellung einer Verbindungsstelle für eine elektrische Verdrahtung.

Die EP 0 192 779 B1 zeigt einen ähnlichen Druckmeßwandler mit einer Stecker/Buchsen-Verbindung für den Anschluß eines wiederverwendbaren elektrischen Kabels sowie einer im Kabel angeordneten Abgleichkarte zum Abgleich der elektrischen Widerstände des Druckmeßwandlers.

Die US 4,603,574 zeigt einen Druckmeßwandler, bei dem zum Testen des Druckmeßwandlers parallel zu einem Brückenzweig der Wheatstonschen Meßbrücke ein Testwiderstand geschaltet ist, der durch gleichzeitiges Betätigen zweier elektrische Schalter parallel zu diesem Brückenzweig geschaltet wird, um einen definierten Anzeigewert zu erhalten. Diese beiden Schalter können sowohl am Druckmeßwandler selbst als auch in dem Zuleitungskabel untergebracht sein.

Die DE 36 31 659 A1 zeigt wiederum einen Druckmeßwandler für allgemeine Zwecke mit einer Stift/Buchsen-Verbindung zum Anschluß des elektrischen Kabels. Gleiches gilt auch für das DE-GM 86 01 071.

Die EP 0 366 651 B1 beschreibt einen Druckmeßwandler mit einem kurzen, fest angelöteten Kabel, das zusammen mit dem Druckmeßwandler nach Gebrauch fortgeworfen wird. Dieses Kabel hat eine Steckverbindung zur Verbindung mit einem weiteren Kabel, wobei dieses weitere Kabel, das dann wiederverwendbar sein soll, einen elektrischen Schalter und einen Nebenschlußwiderstand enthält, der bei Drücken des Schalters parallel zu einem Brückenzweig geschaltet wird, um einen definierten Ablesewert zu erzeugen.

Die Zeitschrift Health Devices, March 1988, Vol. 17, No. 3, Seite 75-94 beschreibt weitere einmal verwendbare Druckmeßwandler, denen allen gemeinsam ist, daß am Druckmeßwandler ein kurzes Kabelstück fest angebracht ist, das über eine Steckkupplung mit einem zum Monitor führenden Kabel verbindbar ist.

Aus dem DE-GM 89 08 951 ist schließlich ein Drucksensor mit integriertem Steckergehäuse bekannt.

Aufgabe der Erfindung ist es, den Druckmeßwandler der eingangs genannten Art dahingehend zu verbessern, daß er bei einfachem und kompaktem Aufbau leicht und kostengünstig zu montieren ist, wenig nur einmal verwendbare Teile aufweist und eine einfache und sichere Handhabung gestattet, insbesondere auch eine einfache und sichere elektrische Verbindung mit einem zum Monitor oder sonstigen Anzeigegerät führenden Kabel aufweist.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Kurz zusammengefaßt weist der einmal verwendbare Teil des Druckmeßwandlers nach der Erfindung nur noch ein Gehäuse und ein mit elektrischen Schaltkreisen und einem Druckmeßelement bestücktes Substrat auf. Das elektrische Kabel wird durch einen besonderen Steckverbinder angeschlossen, der unmittelbar, d.h. ohne Zwischenschaltung weiterer Kabelabschnitte, Kontaktflächen des Substrates kontaktiert und dabei das Substrat und einen Gehäusedeckel zangenartig übergreift und damit die Kontaktierung weitestgehend frei von mechanischen Beanspruchungen des Substrates herstellt.

Das Substrat ist nach einer Ausgestaltung der Erfindung nur in das Gehäuse eingelegt und wird dort durch einen vorzugsweisen lösbaren Deckel gehalten.

Nach einer Weiterbildung der Erfindung hat der Deckel eine Öffnung für den Durchtritt einer Zunge des Steckverbinders. Damit umfaßt der Steckverbinder das Substrat und den Deckel zangenartig. Da der Deckel vollflächig an dem Substrat anliegt, werden alle von dem Steckverbinder aufgebrachten mechanischen Kräfte von ihm auch wieder abgefangen.

Die erste Kammer, die das Druckmeßelement enhält, wird durch einen Abschnitt des Substrates begrenzt und durch einen gegen das Substrat drückenden Dichtungsring flüssigkeitsdicht isoliert. Zusätzlich ist an dem Deckel ein im wesentlichen kongruent zu dem genannten Dichtungsring angeordneter Dichtungsring vorhanden, der gegen die der ersten Kammer abgewandte Seite des Substrates drückt und damit auch eine durch das Substrat hindurchgehende Öffnung zum Abgleich bzw. zur Eichung des Druckmeßwandlers abdichtet.

Die Flüssigkeitsanschlüsse und ein Spülsystem sind einstückig in das Gehäuse des Druckmeßwandlers integriert. Ebenso ist eine Prüftaste in dem Gehäuse integriert, die in einer Gehäuseöffnung gehalten und geführt ist, an ihrer dem Substrat zugewandten Seite mindestens eine Schaltkontaktfläche aufweist und die mit auf dem Substrat aufgedruckten Schaltkontaktflächen zusammenwirkt, um das System zu prüfen. Diese Taste ist aus gummi-elastischem Material, wobei die Schaltkontaktflächen so angebracht sind, daß aufgrund der Federwirkung des gummi-elastischen Materiales bei Nichtbetätigen der Taste kein Kontakt zu den auf dem Substrat vorhandenen Schaltkontaktflächen hergestellt ist. Damit wird auch im Gegensatz zum bisherigen Stand der Technik keine separate Feder für die Taste benötigt.

Nach einer Weiterbildung der Erfindung sind der Druckmeßwandler und der Steckverbinder an einer Halteplatte fixierbar, die diese beiden Teile in korrekter Beziehung zueinander ausrichtet und ein Kuppeln derselben gestattet.

Im folgenden wird die Erfindung anhand eines Ausführungsbeispieles in Zusammenhang mit der Zeichnung ausführlicher erläutert. Es zeigt:
- Fig. 1: Eine Draufsicht auf den Druckmeßwandler nach der Erfindung, der an einer Halteplatte fixiert ist;
- Fig. 2: einen Schnitt längs der Linie I-I der Fig. 1;
- Fig. 3: einen Schnitt längs der Linie II-II der Fig. 1;
- Fig. 4: eine Ansicht der Unterseite des Druckmeßwandlers bei geöffnetem Gehäuse und entnommenem Substrat;
- Fig.5: eine Ansicht der Außenseite eines das Gehäuse des Druckmeßwandlers verschließenden Deckels;
- Fig. 6: eine Stirnansicht des Deckels der Fig. 5, gesehen in Richtung des Pfeiles X der Fig. 5;
- Fig. 7: eine Draufsicht auf das bei dem Druckmeßwandler verwendete Substrat mit aufgebrachtem Druckmeßelement;
- Fig. 8: eine geschnittene Seitenansicht eines mit dem Druckmeßwandler verbindbaren Steckers;
- Fig. 9: eine Draufsicht auf die Unterseite des Steckers der Fig. 8;
- Fig. 10: eine Stirnansicht des Steckers der Fig. 8, gesehen in Richtung des Pfeiles Y der Fig. 8;
- Fig. 11: eine Draufsicht auf eine Halteplatte zur Fixierung des Druckmeßwandlers und des Steckers;
- Fig. 12: einen Schnitt längs der Linie III-III der Fig. 11; und
- Fig. 13: eine Vorderansicht des Druckmeßwandlers ohne Steckverbinder.

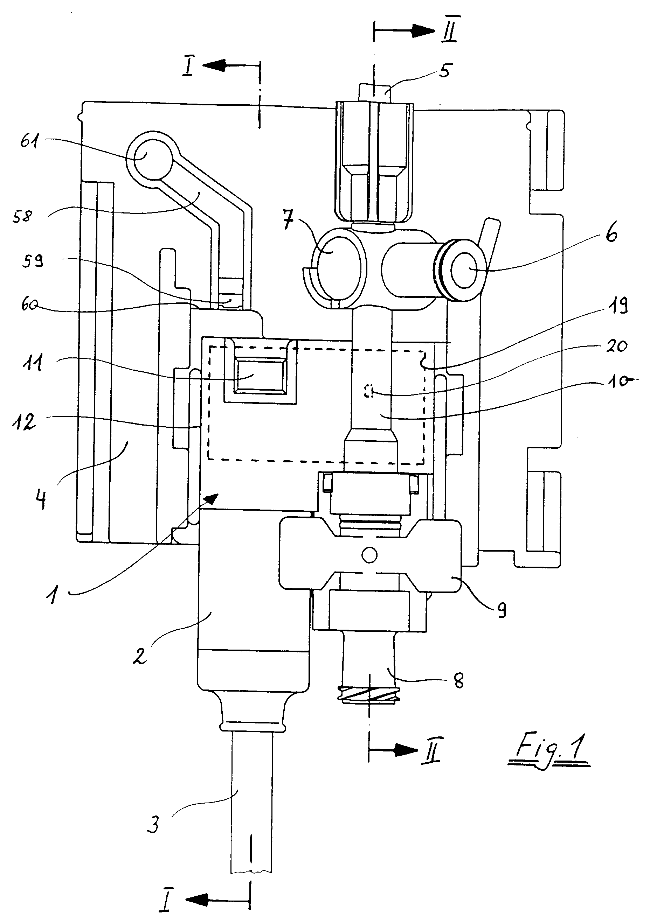

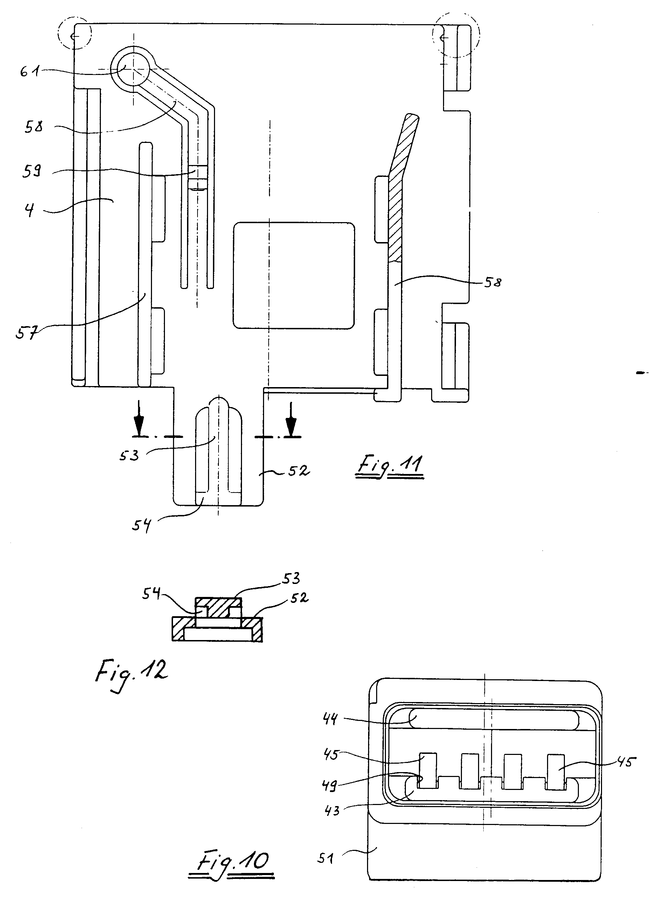

Der Druckmeßwandler, der in seiner Gesamtheit mit dem Bezugszeichen 1 bezeichnet ist, wird über einen Steckverbinder 2 elektrisch mit einem Kabel 3 verbunden, das üblicherweise an einen Monitor angeschlossen ist. Die Einheit aus Druckmeßwandler 1 und Steckverbinder 2 ist während des Einsatzes an einer Halteplatte 4 befestigt, wobei - wie im Zusammenhang mit den Fig. 9 - 12 noch näher beschrieben wird - der Druckmeßwandler 1 und der Steckverbinder 2 jeweils separat an der Halteplatte 4 fixierbar sind, so daß auch der Kupplungsvorgang im Zusammenwirken mit der Halteplatte erfolgen kann. Der Druckmeßwandler hat einen ersten Anschluß 5 für Flüssigkeit, der normalerweise über einen Schlauch mit einem Blutgefäß des Patienten verbunden ist. Ein zweiter Anschluß 6 für Flüssigkeit dient zur Zufuhr einer weiteren Flüssigkeit, wzb. einer Kochsalzlösung, die zum einem dazu dient, das System anfänglich zu füllen und zu anderen dazu, ein Verstopfen des Systems durch koakuliertes Blut zu verhindern. Zwischen beiden Anschlüssen 5 und 6 ist ein 3-Wege-Ventil vorgesehen, wobei in Fig. 1 eine Öffnung 7 für ein Ventilküken 7' (Fig. 3) zu erkennen ist. Weiter weist der Druckmeßwandler eine Öffnung 8 auf, die über ein Spülventil 9 an ein den Öffnungen 5 und, 6 abgewandtes Ende einer Druckmeßleitung 10 angeschlossen ist. Dieses Ventil 9 und die Öffnung 8 dienen dazu, das System zu spülen und insbesondere beim anfänglichen Füllen Luftblasen entweichen zu lassen. Die Druckmeßleitung 10 ist integrierter Bestandteil des Druckmeßwandlers 1 und leitet die Flüssigkeit, deren Druck gemessen werden soll, zu einer weiter unten beschriebenen Druckmeßkammer.

Der Druckmeßwandler 1 weist eine Prüftaste 11 auf, die im Gehäuse 12 des Druckmeßwandlers geführt ist und die einen elektrischen Schalter betätigt, der eine Funktionsprüfung des Druckmeßwandlers ermöglicht. Wie im Stand der Technik bekannt, wird mittels dieses Schalters ein Brückenzweig einer Meßbrücke in definierter Weise verändert, beispielsweise durch Parallelschaltung eines Testwiderstandes über einen Brückenzweig, worauf ein definierter Anzeigewert von beispielsweise 100 mm/Hg am Monitor abzulesen ist, wenn der Druckmeßwandler einwandfrei funktioniert und alle elektrischen Verbindungen korrekt hergestellt sind.

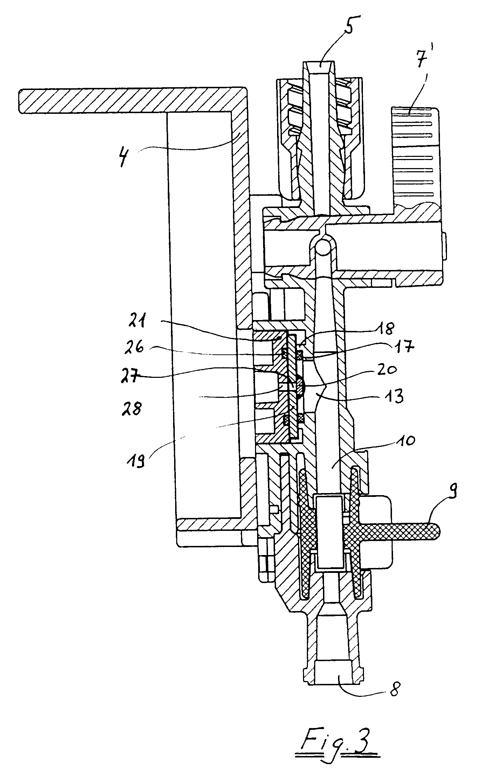

Im Inneren des Druckmeßwandlers sind zwei flüssigkeitsdicht gegeneinander abgetrennte Kammern 13 und 14 (Fig. 4) vorgesehen, wobei die erste Kammer 13 die "Meßkammer" ist, die mit der Druckmeßleitung 10 in Flüssigkeitsverbindung steht, während die zweite Kammer 14 für die Herstellung der elektrischen Anschlüsse zwischem dem Steckverbinder 2 und einem Druckmeßelement verwendet wird. Diese beiden Kammer sind durch einen Steg 15 (Fig. 4) voneinander getrennt, wobei zum besseren Verständnis darauf hingewiesen wird, daß dieser Steg im vorliegenden Ausführungsbeispiel keine Dichtungsfunktion zur flüssigkeitsdichten Abtrennung der beiden Kammern 13 und 14 übernimmt.

Die Druckmeßkammer 13 steht über eine Öffnung 16 mit der Druckmeßleitung 10 in Verbindung, wobei rings um diese Öffnung 16 ein Dichtungsring 17 in Form eines Silikon-O-Ringes angeordnet ist, der von einer zylindrischen Führung 18 in seiner Position gehalten ist.

Im Inneren des Druckmeßwandlers befindet sich ein Substrat 19, das hier ein Keramikplättchen ist (vgl. Fig. 7) und das ein Druckmeßelement 20 trägt, welches sich im Bereich der Kammer 13 befindet. Dieses Substrat ist in das Innere des Druckmeßwandlers 1 eingelegt und stützt sich an dem Dichtungsring 17 ab, so daß der Dichtungsring 17 und der von diesem eingeschlossene Bereich des Substrates die flüssigkeitsdichte Abdichtung der Meßkammer 13 übernehmen. Auf der der Meßkammer 13 abgewandten Seite des Substrates ist ein Gehäusedeckel 21 vorhanden, der in eine im wesentlichen rechteckige Gehäuseöffnung 22 einsetzbar und dort lösbar fixiert werden kann. Im Bereich dieser Öffnung sind vier Abstützungen 23 (Fig. 4) vorgesehen, auf denen sich das Substrat abstützt, welches auf der dazu gegenüberliegenden Seite von dem Deckel 21 in seiner Position gehalten wird.

Im Bereich der zweiten Kammer 14 ist in Fig. 4 eine rechteckige Öffnung 24 zu erkennen, die für den Durchtritt und die Führung der Prüftaste 11 vorgesehen ist.

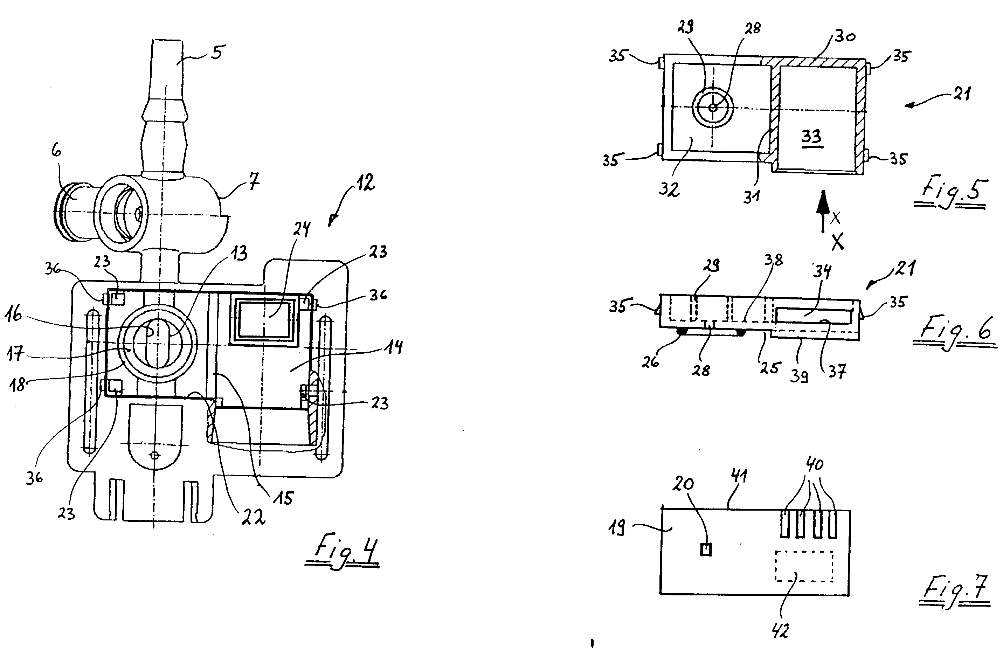

Der dem Substrat 20 zugewandte Boden 25 (Fig. 6) des Deckels 21 ist vollkommen eben, so daß sich das Substrat 21 vollflächig an ihm abstützt. Im Boden 25 des Deckels 21 ist eine Ringnut eingelassen, welche einen weiteren Dichtungsring 26 aufnimmt, der im wesentlichen kongruent zu dem Dichtungsring ist 17, so daß der der Kammer 13 zugeordnete Bereich des Substrates durch die Dichtungsringe 17 und 26 abgedichtet ist. An der Stelle des Substrates, an der das Druckmeßelement 20 aufgebracht ist, befindet sich eine kleine Bohrung 27, die mit einer entsprechenden Bohrung 28 im Gehäusedeckel 21 fluchtet. Rings um die Öffnung 28 weist der Gehäusedeckel 21 einen zylindrischen Anschluß 29 auf, an den ein Schlauch angeschlossen werden kann, um der der Meßkammer 13 abgewandten Seite des Druckmeßelementes 20 einen Prüfdruck zuführen zu können. Hierüber kann eine genaue Eichung mit einem definierten Referenzdruck vorgenommen werden und gleichzeitig während des normalen Meßbetriebes der Rückseite des Druckmeßelementes Atmosphärendruck als Referenzdruck zugeführt werden.

Wie am besten aus Fig. 4 zu erkennen ist, wird also das Substrat 21 in die Gehäuseöffnung 22 eingesetzt und stützt sich dabei an den Abstützungen 23 und dem Dichtungsring 17 und gegebenenfalls auch noch an dem Steg 15 ab. Anschließend wird der Deckel 21 eingesetzt, der mit seinem Dichtungsring 26 und im übrigen mit seinem ebenen Boden 25 an der Rückseite des Substrates anliegt und dieses in Position hält. Das Druckmeßelement 20 des Substrates 19 liegt dabei der Meßkammer 13 zugewandt in der Meßkammer.

Der Deckel 21 (vgl. Fig. 5 und 6) hat - wie erwähnt - einen ebenen Boden 25, von dem senkrechte, einen Rahmen 30 bildende Wände abstehen, sowie einen in zusammengesetzter Stellung parallel zum Steg 15 liegenden Steg 31. Beidseitig dieses Steges 31 bildet der Rahmen 30 zwei rechteckige Bereiche 32 und 33, die der ersten bzw. zweiten Kammer 13 bzw. 14 des Druckmeßwandlers gegenüberliegen. Die beiden Bereiche 32 und 33 haben etwas unterschiedliche Abmessungen, um zu gewährleisten, daß der Deckel nur in einer definierten Position eingesetzt werden kann. Entsprechend ist auch die Öffnung 22 an diese Form angepaßt.

An zwei gegenüberliegenden Wänden des Rahmens 30 sind Rastvorsprünge 35 angeformt, die in Rastausnehmungen 36 im Gehäuse 12 eingreifen und damit den Deckel gegenüber dem Gehäuse verriegeln. Mittels eines Werkzeuges läßt sich der Deckel jedoch ohne weiteres entfernen, so daß bei der Entsorgung eines gebrauchten Druckmeßwandlers das aus Kunststoff bestehende Gehäuse und das Substrat mit metallenen Leiterbahnen und dem Druckmeßelement leicht voneinander getrennt werden können. Dies ist im Hinblick auf die wachsenden Umweltanforderungen von großer Bedeutung.

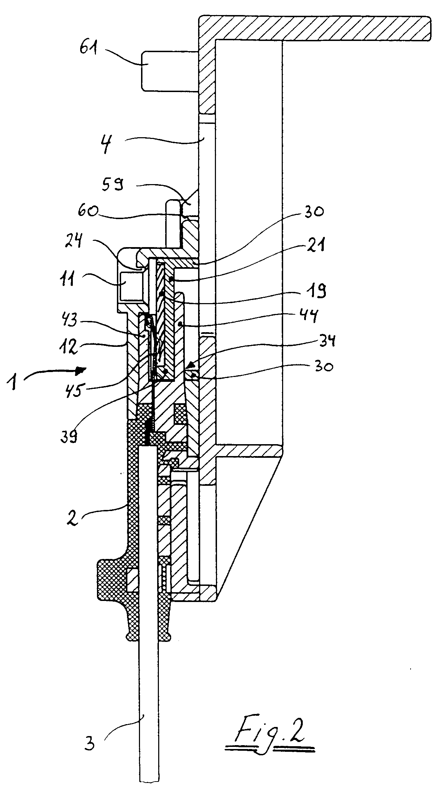

Der Deckel 21 weist an einer Stirnseite des Rahmens 30 eine rechteckige Öffnung 34 auf, deren eine Kante 37 mit der dem Boden 25 abgewandten Seite 38 des Deckels fluchtet. Diese Öffnung 34 dient für den Durchtritt einer Zunge des Steckverbinders 2, was insbesondere in Zusammenhang mit Fig. 2 noch ausführlich erläutert wird. Diese Öffnung 34 ist im Bereich 33 des Deckels angeordnet und damit mit der zweiten Kammer 14 ausgerichtet. Im Bereich dieser Öffnung 34 steht die entsprechende Seitenwand 39 über den Boden 25 des Deckels 21 vor, so daß dieser Wandabschnitt einen verbreiterten Anschlag für das Einführen des Steckverbinders 2 bildet.

Wie aus Fig. 7 zu erkennen ist, weist das Substrat auf der in Fig. 7 zu sehenden Oberseite, die im montierten Zustand in Richtung der Meßkammer 13 weist, das Druckmeßelement 20 und mehrere Kontaktflächen 40 auf, die seitlich an einen Rand 41 des Substrates 19 herausgeführt sind. Diese Kontaktflächen 40 sind mit nicht dargestellten, aufgedruckten Leiterbahnen verbunden, die zu dem Druckmeßelement 20 und sonstigen, nicht dargestellten externen elektrischen Beschaltungen für das Druckmeßelement 20 verbunden sind. Weiterhin ist in Fig. 7 durch ein gestricheltes Kästchen 42 ein Bereich markiert, der der Prüftaste 11 (Fig. 1) gegenüberliegt und der nicht dargestellte Schaltkontaktflächen aufweist, die durch Betätigen der Prüftaste 11 elektrisch miteinander verbunden werden, um die Prüffunktion auszuführen. Die Kontaktflächen 40 sind in dem Bereich des Substrates 19 aufgebracht, der bei zusammengesetztem Druckmeßwandler in der zweiten Kammer 14 liegt, wobei die Kontaktflächen 40 auf der dem Deckel 21 abgewandten, d.h. dem Inneren des Gehäuses zugewandten Seite liegen.

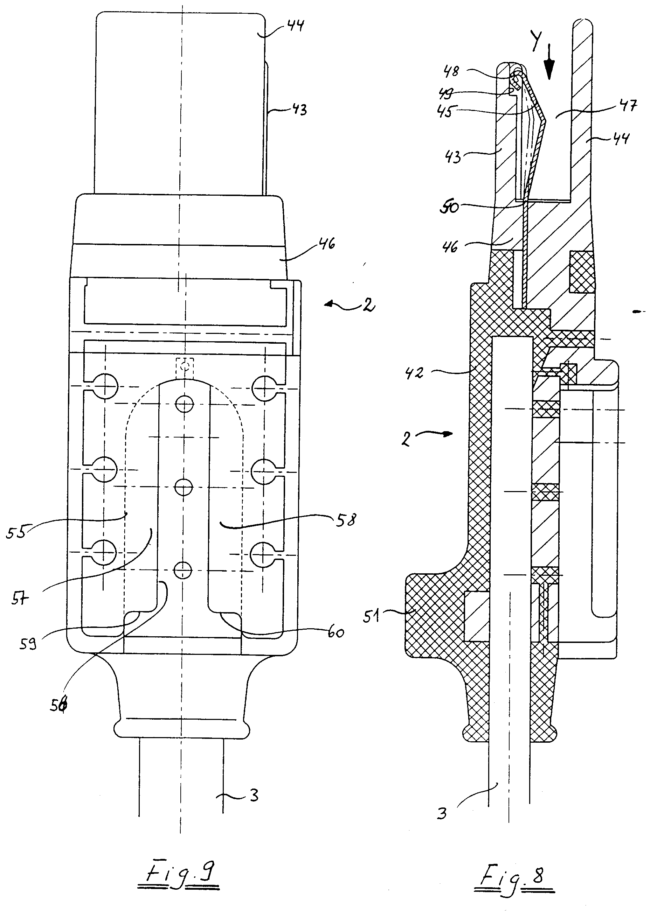

Fig. 8 zeigt detaillierter den Steckverbinder 2, der ein Steckergehäuse 42 und an der in Einschieberichtung nach vorne weisenden Seite 2 parallel im Abstand zueinander verlaufende Zungen 43 und 44 aufweist, wobei zwischen diesen beiden Zungen elektrische Federkontakte 45 angeordnet sind, die bei eingestecktem Steckverbinder die Kontaktflächen 40 des Substrates kontaktieren. Die Federkontakte 45 sind fest in einem Gehäuseabschnitt 46 verankert und erstrecken sich in den Zwischenraum 47 zwischen den beiden Zungen 43 und 44. Das freie Ende 48 der Federkontakte 45 ist hier hakenförmig umgebogen und stützt sich an einer zum Zwischenraum 47 weisenden Ausnehmung 49 der Zunge 43 ab. Zwischen der Stelle 50, an der die Federkontakte 45 fest eingespannt sind und ihrem freien Ende 48 sind die Federkontakte von der Zunge 43 fortweisend und sich in den Zwischenraum 47 erstreckend aufgebogen, so daß sie durch Federwirkung die erforderliche Andruckkraft gegenüber den Kontaktflächen 40 des Substrates 19 aufbringen.

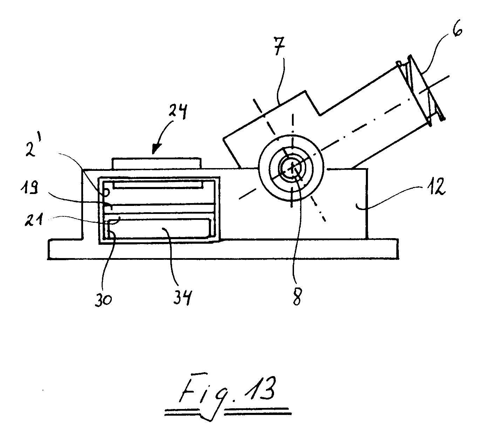

Die Federkontakte 45 sind in nicht dargestellter Weise mit zugeordneten Adern des Kabels verbunden, beispielsweise durch Anlöten oder Kabelklemmschuhe, wobei das Kabel 3 in das Innere des Steckergehäuses 42 eingeführt und dort gehalten ist, beispielsweise dadurch, daß das Steckergehäuse 42 in Spritzgußtechnik um das Kabel 3 herum gespritzt wird. Das Steckergehäuse 42 ist aus elastischem Kunststoffmaterial, was durch die doppelte Schraffur in Fig. 3 und 8 angedeutet ist. Der Gehäuseabschnitt 46 mit den beiden Zungen 43 und 44 ist dagegen aus härterem Material, das sich an der Unterseite (Fig. 9) auch über weitere Teile des Steckergehäuses 42 erstreckt und - wie im Zusammenhang mit Fig. 11 erläutert wird - zur Halterung des Steckverbinders an der Halteplatte 4 (Fig. 1) dient.

Wie am besten aus Fig. 2 und 13 zu erkennen ist, weist das Gehäuse 12 des Druckmeßwandlers 1 eine rechteckige Öffnung 2' auf, in die die beiden Zungen 43 und 44 und ein sich daran anschließender Teil des Gehäuseabschnittes 46 eingeführt werden kann. Die beiden Zungen 43 und 44 umgreifen dabei den entsprechenden Abschnitt des Substrates 19 und des Gehäusedeckels 21, wobei die Zunge 44 durch die rechteckige Öffnung 34 des Gehäusedeckels eingeführt ist. Die Zunge 43 mit den Federkontakten 45 greift damit in die zweite Kammer 14 des Druckmeßwandlers ein, während die Zunge 44 außerhalb des Gehäuses des Druckmeßwandlers liegt. Dadurch daß die beiden Zungen 43 und 44 das Substrat 19 und dem Gehäusedeckel 21 zangenartig umgreifen, werden diese Teile von den beiden Zungen 43 und 44 abgestützt und von den Federkontakten 44 auf das Substrat ausgeübte Druckkräfte werden nicht nur von den Rastvorsprüngen 45 des Gehäusedeckels abgefangen, sondern auch von der Zunge 44. Damit erhält man eine von mechanischen Spannungen weitestgehend freie Halterung des (Keramik)-Substrates 19 im Gehäuse des Druckmeßwandlers.

Da die Federkontakte 45 die Kontaktflächen 40 auf dem Substrat unmittelbar berühren, entfallen in dem einmal zu verwendenden Druckmeßwandler jegliche zusätzlichen Verdrahtungen, die bei den früheren Druckmeßwandlern benötigt wurden. Hierdurch ist zum einen die Herstellung vereinfacht und verbilligt, wodurch sich auch beim Anwender die Kosten des nur einmal verwendbaren Druckmeßwandlers verringern und zum anderen ist die spätere Entsorgung vereinfacht, da keine vom Kunststoff zu trennende Metallteile vorhanden sind, mit Ausnahme der auf dem Substrat aufgedruckten elektrischen Leiterbahnen, die - wie oben erwähnt - von dem Kunststoff des Gehäuses dadurch leicht getrennt werden können, daß der Deckel 21 entfernt und das Substrat herausgenommen wird.

Das Ankuppeln und Entkuppeln des Steckverbinders mit dem Druckmeßwandler kann sehr einfach von Hand erfolgen. Um für das Trennen der Verbindung eine bessere Handhabe zu bekommen, ist am Steckergehäuse ein Vorsprung 51 angebracht, über den die notwendigen Zugkräfte aufgebracht werden können, durch den verhindert wird, daß der Benutzer am Kabel 3 zieht.

Nach einer weiteren Ausgestaltung der Erfindung sind der Steckverbinder 2 und die an sich bekannte Halteplatte 4 so modifiziert, daß das Kuppeln und Halten des Steckverbinders erleichtert ist und die miteinander zu kuppelnden Teile, d.h. der Druckmeßwandler 1 und der Steckverbinders 2 während des Kuppelns und Entkuppelns in ihrer wechselseitigen Ausrichtung korrekt geführt bzw. gehalten sind. Während des Gebrauches werden der Druckmeßwandler und der Steckverbinder in ihrer gegenseitigen Kopplung von der Halteplatte verriegelt gehalten, sodaß ein unbeabsichtigtes Lösen des Steckverbinders, wzb durch Zug am Kabel vermieden wird.

Die aus der nicht vorveröffentlichten Patentanmeldung P 43 17 985.1 an sich bekannte Halteplatte 4 weist zu diesem Zwecke einen über ihre ansonsten rechteckige Kontur hinausragenden Ansatz 52 auf, an dem der Steckverbinder 2 in korrekter Ausrichtung gegenüber dem Druckmeßwandler 1 positioniert und gehalten wird. Der Ansatz 52 liegt in der Ebene der Halteplatte 4 und weist einen von dieser Ebene vorstehenden Verriegelungsvorsprung 53 auf, der einen T-förmigen Querschnitt (vgl. Fig. 12) hat. Am der Halteplatte 4 abgewandten Ende ist der Verriegelungsvorsprung 53 durch eine quer zu dessen Längsrichtung verlaufende Wand begrenzt, die als Anschlag 54 dient.

Der Steckverbinder 2 weist an seiner der Halteplatte 4 zugewandten Unterseite eine entsprechend geformte Ausnehmung 55 auf, in das T-förmige Profil des Verriegelungsvorsprunges 53 eingeschoben werden kann. Diese Ausnehmung ist in Fig. 9 durch eine gestrichelte Linie 55 dargestellt. Die Ausnehmung 55 weist eine in Längsrichtung des Steckverbinders 2 verlaufende Öffnung 56 auf, in welcher der senkrechte Steg des Verriegelungsvorsprunges 53 eingreift. Die beiden einander gegenüberliegenden und durch die Ausnehmung 56 voneinander getrennten Wände 57 und 58 bilden am kabelseitigen Ende Gegenanschläge 59 und 60, die mit der Wand 54 in Anschlag kommen und damit eine Längsverschiebung des Steckverbinders begrenzen. Wie am besten aus Fig. 11 zu erkennen ist, wird der Steckverbinder 2 von der Seite der Halteplatte 4 her auf den Ansatz 52 bzw. den Verriegelungsvorsprung 53 bis zum Anschlag 54 aufgeschoben und ist dann mit der Halteplatte fest verbunden. Anschließend wird der Druckmeßwandler 1 auf die Halteplatte aufgeschoben und zwischen zwei Führungen 57 und 58 gehalten. Bei dieser Bewegung erfolgt dann automatisch das Kuppeln des Steckverbinders, bis die in Fig. 1 dargestellte Endstellung erreicht ist. In der Halteplatte 4 ist ein Federarm 58 mit einem Verriegelungsvorsprung 59 vorgesehen, wobei dieser Verriegelungsvorsprung 59 hinter einer Kante 60 des Druckmeßwandlers einrastet und diesen gegen ein Herausziehen sichert. Damit ist die Elektrosteckverbindung zwischen dem Steckverbinder 2 und dem Druckmeßwandler 1 in allen Richtungen fixiert und kann sich nicht unfreiwillig lösen. Zum Entkoppeln muß der Fedearm 58 an einem Druckknopf 61 soweit niedergedrückt werden, daß der Verriegelungsvorsprung 59 die Kante 60 freigibt, worauf der Druckmeßwandler 1 von der Halteplatte herausgezogen werden kann.

## Patentansprüche

1. Druckmeßwandler (1) zur Messung des Druckes einer Flüssigkeit, insbesondere zur invasiven Blutdruckmessung, mit einem Gehäuse (12), das zwei flüssigkeitsdicht voneinander abgetrennte Kammern (13, 14) aufweist, von denen die erste ein Druckmeßelement (20) enthält, das mit dem zu messenden Druck der Flüssigkeit beaufschlagbar ist und von denen die zweite elektrische Verbindungsmittel zum Anschluß eines elektrischen Kabels (3) an mit dem Druckmeßelement (20) verbundene elektrische Leiter aufweist, wobei
das Druckmeßelement (20) an einem flachen Substrat (19) befestigt ist,
die elektrischen Leiter auf dem Substrat (19) aufgebracht sind und seitlich zu Kontaktstellen (40) an den Rand (41) des Substrates (19) geführt sind,
die zweite Kammer (14) des Gehäuses (12) über eine Öffnung (2') zur Aufnahme eines Steckverbinders (2) von der Außenseite des Gehäuses her zugänglich ist,
**dadurch gekennzeichnet**,
daß das Substrat (19) mittig in der Öffnung (2') liegt, so daß beide in der zweiten Kammer (14) liegenden Seiten des Substrates (19) von der Öffnung (2') her zugänglich sind,
daß die elektrischen Verbindungsmittel einen Steckverbinder (2) aufweisen, der zwei parallele Zungen (43, 44) aufweist, zwischen denen das Substrat (19) aufnehmbar ist, wobei eine Zunge (43) an der der anderen Zunge (44) zugewandten Seite elektrische Federkontakte (45) aufweist, die mit den Kontaktstellen (40) des Substrates (19) in Berührung bringbar sind,
daß das Substrat (19) von einem parallel zu ihm liegenden Gehäusedeckel (21) gehalten ist und
daß die parallelen Zungen (43, 44) den Gehäusedeckel (21) und das Substrat (19) zwischen sich einschließen.

2. Druckmeßwandler nach Anspruch 1,
**dadurch gekennzeichnet**,
daß der Gehäusedeckel (21) lösbar an dem Gehäuse (12) befestigt ist.

3. Druckmeßwandler nach Anspruch 1 oder 2,
**dadurch gekennzeichnet**,
daß der Gehäusedeckel (21) an seiner zur Öffnung (2') weisenden Stirnseite eine Ausnehmung (34) für den Durchtritt der Zunge (44) des Steckverbinders (2) aufweist.

4. Druckmeßwandler nach einem der Ansprüche 1 bis 3,
**dadurch gekennzeichnet**,
daß der Gehäusedeckel (21) einen ebenen Boden (25) aufweist, der in montiertem Zustand an dem Substrat (19) anliegt.

5. Druckmeßwandler nach einem der Ansprüche 1 bis 4,
**dadurch gekennzeichnet**,
daß die erste Kammer (13) durch einen das Substrat (19) berührenden, rings um das Druckmeßelement (20) verlaufenden Dichtungsring (17) abgedichtet ist.

6. Druckmeßwandler nach Anspruch 5,
**dadurch gekennzeichnet**,
daß im Boden (25) des Gehäusedeckels (21) ein im wesentlichen kongruent zu dem Dichtungsrichtung (17) verlaufender weiterer Dichtungsring (26) eingelassen ist, der gegen die der ersten Kammer (13) abgewandte Seite des Substrates drückt.

7. Druckmeßwandler nach einem der Ansprüche 1 bis 5,
**dadurch gekennzeichnet**,
daß das Substrat (19) lose in das Gehäuse (12) eingelegt und nur durch den lösbaren Deckel (21) gegen Herausfallen gesichert ist.

8. Druckmeßwandler nach einem der Ansprüche 1 bis 7,
**dadurch gekennzeichnet**,
daß im Gehäuse (12) des Druckmeßwandlers ein Dreiwegehahn (7) und ein Spülsystem (8, 9) integriert sind.

9. Druckmeßwandler nach einem der Ansprüche 1 bis 8,
**dadurch gekennzeichnet**,
daß das Gehäuse (12) im Bereich der zweiten Kammer (14) gegenüberliegend zum Substrat (19) eine Öffnung (24) aufweist, zum Durchtritt und zur Führung einer Prüftaste (11), die an ihrer dem Substrat (19) zugewandten Seite mindestens einen elektrischen Schaltkontakt trägt, der mit auf dem Substrat (21) aufgebrachten Schaltkontaktflächen zusammenwirkt.

10. Druckmeßwandler nach einem der Ansprüche 1 bis 9,
**dadurch gekennzeichnet**,
daß der Steckverbinder (2) eine Ausnehmung (55) aufweist, über die er an einem Verriegelungsvorsprung (53) einer Halteplatte (4) fixierbar ist und daß die Halteplatte (4) Führungen (57, 58) für die Führung und Halterung des Druckmeßwandlers aufweist, wobei diese Führungen und Verriegelungsvorsprünge so gegeneinander ausgerichtet sind, daß der Steckverbinder und der Druckmeßwandler miteinander koppelbar sind.

## Claims

1. A pressure transducer (1) for measuring the pressure of a fluid, in particular for invasive blood pressure measurement, having a housing (12) with two chambers (13, 14) separated from one another in a manner so as to be fluid-tight in relation to one another, of which the first chamber comprises a pressure measuring element (20) actable upon with the fluid pressure to be measured and of which the second chamber has electrical connecting means for connection of an electric cable (3) to electric conductors connected to the pressure measuring element (20),
the pressure measuring element (20) being secured to a flat substrate (19),
the electrical conductors being put on the substrate (19) and being run laterally - towards the edge (41) of the substrate (19) - to contact points (40),
the second chamber (14) of the housing (12) being accessible from the exterior of the housing via an opening (2') for receiving a connector (2),
characterised in that the substrate (19) lies in the centre of the opening (2'), so that both substrate sides located in the second chamber (14) are accessible from the opening (2'), in that the electrical connecting means have a connector (2) with two parallel tongues (43, 44) between which the substrate (19) can be received, one tongue (43) having electrical spring contacts (45) at the side facing the other tongue (44), which spring contacts (45) can be brought into contact with the contact points (40) of the substrate (19), in that the substrate (19) is held in position by a housing cover (21) located parallel thereto and in that the housing cover (21) and the substrate (19) are enclosed between the parallel tongues (43, 44).

2. A pressure transducer in accordance with Claim 1, characterised in that the housing cover (21) is removably secured to the housing (12).

3. A pressure transducer in accordance with Claim 1 or 2, characterised in that the end face, facing the opening (2'), of the housing cover (21) has a cut-out (34) for passage of the tongue (44) of the connector (2).

4. A pressure transducer in accordance with any one of Claims 1 to 3, characterised in that the housing cover (21) has a plane base (25) which contacts with the substrate (19) in the mounted state.

5. A pressure transducer in accordance with any one of Claims 1 to 4, characterised in that the first chamber (13) is sealed by a gasket seal (17) which contacts with the substrate (19) and which extends around the pressure measuring element (20).

6. A pressure transducer in accordance with Claim 5, characterised in that another gasket seal (26) extending substantially congruent with the gasket seal (17) is let in the base (25) of the housing cover (21), which gasket seal (26) presses against the substrate side facing the first chamber (13).

7. A pressure transducer in accordance with any one of Claims 1 to 5, characterised in that the substrate (19) is loosely inserted into the housing (12) and is only secured against falling out by the removable cover (21).

8. A pressure transducer in accordance with any one of Claims 1 to 7, characterised in that a three-way tap (7) and a rinsing system (8, 9) are integrated in the housing (12) of the pressure transducer.

9. A pressure transducer in accordance with any one of Claims 1 to 8, characterised in that in the region of the second chamber (14), the housing (12) has - opposite the substrate (19) - an opening (24) for the passage and guiding of a test key (11) bearing at least one electrical switching contact at its side facing the substrate (19), which switching contact co-operates with switching contact surfaces put on the substrate (21[sic]).

10. A pressure transducer in accordance with any one of Claims 1 to 9, characterised in that the connector (2) has a cut-out (55) via which it can be fixed to a locking projection (53) of a holding plate (4), and in that the holding plate (4) has guides (57, 58) for guiding and holding the pressure transducer, these guides and locking projections being orientated such in relation to one another that the connector and the pressure transducer can be coupled to one another.

## Revendications

1. Transducteur de pression (1) pour mesurer la pression d'un liquide, en particulier pour la mesure invasive de la pression artérielle, avec un boîtier (12) présentant deux chambres (13, 14) séparées l'une de l'autre de manière étanche aux liquides, dont la première contient un élément de mesure de la pression (20) pouvant être exposé à la pression de liquide qui doit être mesurée et dont la deuxième présente des moyens de connexion électriques destinés à raccorder un câble électrique (3) à des conducteurs électriques reliés à l'élément de mesure de la pression (20), dans lequel
l'élément de mesure de la pression (20) est fixé sur un substrat (19) plat,
les conducteurs électriques sont appliqués sur le substrat (19) et passés latéralement en direction de points de contact (40) situés sur le bord (41) du substrat (19),
la deuxième chambre (14) du boîtier (12) est accessible de l'extérieur du boîtier par une ouverture (2') destinée à recevoir un connecteur enfichable (2),
**caractérisé en ce que,**
le substrat (19) se trouve disposé au centre de l'ouverture (2'), de telle sorte que les deux faces du substrat (19) situées dans la deuxième chambre (14) soient accessibles à partir de l'ouverture (2'),
en ce que les moyens de connexion électriques comprennent un connecteur enfichable (2) qui présente deux languettes parallèles (43, 44), entre lesquelles le substrat (19) peut être inséré, une languette (43) présentant, sur la face orientée vers l'autre languette (44), des contacts électriques à ressort (45) qui peuvent être mis en contact avec les points de contact (40) du substrat (19),
en ce que le substrat (19) est maintenu par un couvercle de boîtier (21) qui lui est parallèle et
en ce que les languettes parallèles (43, 44) renferment entre elles le couvercle du boîtier (21) et le substrat (19).

2. Transducteur de pression selon la revendication 1, **caractérisé en ce que** le couvercle de boîtier (21) est fixé de manière amovible au boîtier (12).

3. Transducteur de pression selon la revendication 1 ou 2, **caractérisé en ce que** le couvercle de boîtier (21) présente, sur sa face orientée vers l'ouverture (2'), un évidement (34) destiné au passage de la languette (44) du connecteur enfichable (2).

4. Transducteur de pression selon l'une des revendications 1 à 3, **caractérisé en ce que** le couvercle de boîtier (21) présente un fond plan (25) qui repose, dans l'état monté, sur le substrat (19).

5. Transducteur de pression selon l'une des revendications 1 à 4, **caractérisé en ce que** la première chambre (13) est rendue étanche par un joint d'étanchéité (17) qui touche le substrat (19) et fait le tour de l'élément de mesure de la pression (20).

6. Transducteur de pression selon la revendication 5, **caractérisé en ce qu'**on engage dans le fond (25) du couvercle de boîtier (21) un autre joint d'étanchéité (26) sensiblement de niveau par rapport au joint d'étanchéité (17), qui s'appuie contre la face du substrat orientée à l'opposé de la première chambre (13).

7. Transducteur de pression selon l'une des revendications 1 à 5, **caractérisé en se que** le substrat (19) est disposé sans fixation dans le boîtier (12) et que seul le couvercle amovible (21) l'empêche de tomber.

8. Transducteur de pression selon l'une des revendications 1 à 7, **caractérisé en ce qu'**un robinet à trois voies (7) et un système de rinçage (8, 9) sont intégrés dans le boîtier (12) du transducteur de pression.

9. Transducteur de pression selon l'une des revendications 1 à 8, **caractérisé en ce que** le boîtier (12) présente, au niveau de la deuxième chambre (14), une ouverture (24) faisant face au substrat (19), qui sert au passage et au guidage d'un bouton de contrôle (11) qui porte, sur sa face orientée vers le substrat (19), au moins un contact électrique qui coopère avec des surfaces de contact disposées sur le substrat (21).

10. Transducteur de pression selon l'une des revendications 1 à 9, **caractérisé en ce que** le connecteur enfichable (2) présente un évidement (55) par lequel il peut être fixé sur une saillie de verrouillage (53) d'une plaque de fixation (4) et en ce que la plaque de fixation (4) présente des guides (57, 58) pour le guidage et le maintien du transducteur de pression, ces guides et saillies de verrouillage étant orientés les uns par rapport aux autres de telle sorte que le connecteur enfichable et le transducteur de pression puissent être couplés l'un avec l'autre.
